# EUROPEAN PATENT SPECIFICATION

(11) **EP 0 922 111 B1**
(45) Date of publication and mention of the grant of the patent: **01.12.2004**
(21) Application number: 97930902.8
(22) Date of filing: 23.07.1997
(51) Int. Cl.: C12N 15/86, C07K 16/46, C07K 19/00, C12N 5/10, A61K 39/395, C07K 1/28, C07K 14/705, C12N 15/13, C12N 15/62

(54) **INDUCTION OF T CELL TOLERANCE USING A SOLUBLE MOLECULE THAT CAN SIMULTANEOUSLY BLOCK TWO COSTIMULATORY PATHWAYS**
INDUZIERUNG VON T ZELL TOLERANZ UNTER VERWENDUNG EINES LÖSLICHEN MOLEKÜLS, DASS GLEICHZEITIG ZWEI KOSTIMULIERUNGSWEGE BLOCKIEREN KANN
INDUCTION DE LA TOLERANCE AUX LYMPHOCYTES T AU MOYEN D'UNE MOLECULE SOLUBLE POUVANT BLOQUER SIMULTANEMENT DEUX MECANISMES D'ACTION COSTIMULANTS

(30) Priority: 23.07.1996 US 22070 P
(43) Date of publication of application: 16.06.1999
(73) Proprietor: Tanox Pharma B.V., 1105 BE Amsterdam (NL)
(72) Inventor: DE BOER, Mark, NL-1261 BT Blaricum (NL)
(74) Representative: van Westenbrugge, Andries
(86) International application number: PCT/NL1997/000438
(87) International publication number: WO 1998/003670

(56) References cited:
- WO-A-94/01547
- WO-A-95/08577
- C. LARSEN ET AL.: "Long-term acceptance of skin and cardiac allografts after blocking CD40 and CD28 pathways." NATURE, vol. 381, no. 6581, 30 May 1996, LONDON, GB, pages 434-438, XP002037035 cited in the application
- N. GRIGGS ET AL.: "Contribution of CD28/CTLA4/B7 and gp39/CD40 costimulation pathways in clonal expansion and functional acquisition of self reactive T cells." JOURNAL OF CELLULAR BIOCHEMISTRY, SUPPLEMENT, vol. 0, no. 21 part A, 1995, NEW YORK, NY, USA, page 141 XP002037030
- N. GRIGGS ET AL.: "The relative contribution of the CD28 and gp39 costimulatory pathways in the clonal expansion and pathogenic acquisition of self-reactive T cells." THE JOURNAL OF EXPERIMENTAL MEDICINE, vol. 183, no. 3, 1 March 1996, NEW YORK, NY, USA, pages 801-810, XP002043672
- A. TANG ET AL.: "Suppression of murine allergic contact dermatitis by CTLA4Ig." THE JOURNAL OF IMMUNOLOGY, vol. 157, no. 1, 1 July 1996, BALTIMORE, MD, USA, pages 117-125, XP002037034
- M. DE BOER ET AL.: "Generation of monoclonal antibodies to human lymphocyte cell surface antigens using insect cells expressing recombinant proteins." JOURNAL OF IMMUNOLOGICAL METHODS, vol. 152, no. 2, 10 August 1992, AMSTERDAM, NL, pages 15-23, XP002043673

### SEQUENCE LISTING

(1) GENERAL INFORMATION:
   (i) APPLICANT:
      (A) NAME: Pangenetics B.V.
      (B) STREET: Paasheuvelweg 15
      (C) CITY: Amsterdam
      (E) COUNTRY: The Netherlands
      (F) POSTAL CODE (ZIP): 1105 BE
   (ii) TITLE OF INVENTION: Compositions and methods for induction of T cell tolerance
   (iii) NUMBER OF SEQUENCES: 22
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA: APPLICATION NUMBER: WO PCT/NL97/00438
(2) INFORMATION FOR SEQ ID NO: 1:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 1:
   GCGCAGGCTT GGCCCAGCCG GCCATGGCCC AGGTSMARCT GCAGSAGTCT GG 52
(2) INFORMATION FOR SEQ ID NO: 2:
   (i) SEQUENCE CHARACTERISTICS:
      (A) LENGTH: 89 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 2:
   GTGAGCTCGA TGTCCGATCC GCCACCGCCA GAGCCACCTC CGCCTGAACC GCCTCCACCT 60
   GAGGAGACGG TGACCGTGGT CCCTTGGCC 89
(2) INFORMATION FOR SEQ ID NO: 3:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 3:
   GACATCGAGC TCACYCAGTC TCCA 24
(2) INFORMATION FOR SEQ ID NO: 4:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 4:
(2) INFORMATION FOR SEQ ID NO: 5:
   (i) SEQUENCE CHARACTERISTICS:
      (A) LENGTH: 339 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 5:
   CAGGTGCAAC TCGTGGAGTC TGGACCTGGC CTGGTGAAAC CCTCACAGAG CCTGTCCATC 60
   ACATGCACTG TCTCTGGGTT CTCATTATCC AGATATAGTG TATACTGGGT TCGCCAGCCT 120
   CCAGGAAAGG GTCTGGAGTG GCTGGGAATG ATGTGGGGTG GTGGATCCAC AGACTATAAT 180
   TCAGCTCTCA AATCCAGACT GACCATCAGC AAGGACACCT CGAAGAACCA GGTCTTCTTA 240
   AAAATGAACA GTCTGCGAGC TGAGGACACA GCCATGTACT ACTGTGTCAG AACCGATGGG 300
   GACTACTGGG GTCAAGGAAC CACCGTCACC GTCTCCTCA 339
(2) INFORMATION FOR SEQ ID NO: 6:
   (i) SEQUENCE CHARACTERISTICS:
      (A) LENGTH: 339 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 6:
   GACCTCCAGC TGACCCAGTC TCCACTCTCC CTGCCTGTCA GTCTTGGAGA TCGAGCCTCC 60
   ATCTCTTGCA GATCTAGTCA GAGCCTTGTA AACAGTAATG GAAACACCTA TTTACATTGG 120
   TACCTGCAGA AGCCAGGCCA GTCTCCAAAG CTCCTGATCT ACAAAGTTTC CAACCGATTT 180
   TCTGGGGTCC CAGACAGGTT CAGTGGCAGT GGATCAGGGA CAGATTTCAC ACTCAAGATT 240
   AGCAGAGTGG AGGCTGAGGA TGTGGGAGTT TATTACTGCT CTCAAAGTAC ACATGTTCCG 300
   TGGACGTTCG GTGGAGGCAC CAAGCTGGAA ATCAAACGA 339
(2) INFORMATION FOR SEQ ID NO: 7:
   (i) SEQUENCE CHARACTERISTICS:
      (A) LENGTH: 723 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 7:
   CAGGTGCAGC TGCAGGAGTC TGGACCTGGC CTGGTGAAAC CCTCACAGAG CCTGTCCATC   60
   ACATGCACTG TCTCTGGGTT CTCATTATCC AGATATAGTG TATACTGGGT TCGCCAGCCT  120
   CCAGGAAAGG GTCTGGAGTG GCTGGGAATG ATGTGGGGTG GTGGATCCAC AGACTATAAT  180
   TCAGCTCTCA AATCCAGACT GACCATCAGC AAGGACACCT CGAAGAACCA GGTCTTCTTA  240
   AAAATGAACA GTCTGCGAGC TGAGGACACA GCCATGTACT ACTGTGTCAG AACCGATGGG  300
   GACTACTGGG GCCAAGGGAC CACGGTCACC GTCTCCTCAG GTGGAGGCGG TTCAGGCGGA  360
   GGTGGCTCTG GCGGTGGCGG ATCGGACATC GAGCTCACTC AGTCTCCACT CTCCCTGCCT  420
   GTCAGTCTTG GAGATCGAGC CTCCATCTCT TGCAGATCTA GTCAGAGCCT TGTAAACAGT  480
   AATGGAAACA CCTATTTACA TTGGTACCTG CAGAAGCCAG GCCAGTCTCC AAAGCTCCTG  540
   ATCTACAAAG TTTCCAACCG ATTTTCTGGG GTCCCAGACA GGTTCAGTGG CAGTGGATCA  600
   GGGACAGATT TCACACTCAA GATTAGCAGA GTGGAGGCTG AGGATGTGGG AGTTTATTAC  660
   TGCTCTCAAA GTACACATGT TCCGTGGACG TTCGGTGGAG GCACCAAGCT GGAAATAAAA  720
   CGG                                                                723
(2) INFORMATION FOR SEQ ID NO: 8:
   (i) SEQUENCE CHARACTERISTICS:
      (A) LENGTH: 375 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 8:
   GAGGTCCAAC TGCAGCAGTC TGGACCTGAG CTGGAGAAGC CTGGCGCTTC AGTGAAGATA 60
   TCCTGCAAGG CTTCTGGTTA CTCATTCACT GACTACAACA TGAACTGGGT GAAGCAGAGC 120
   AATGGAAAGA GCCTTGAGTG GATTGGAAAT ATTGATCCTT ACTATGGTGG TACTAGTTAC 180
   AATCAGAAGT TCAAGGGCAA GGCCACATTG ACTGTAGACA AATCCTCCAG CACAGCCTAC 240
   ATGCAGCTCA ACAGCCTGAC ATCTGAAGAC TCTGCAGTCT ATTTCTGTGC AAGATGGGAC 300
   TATAGGTACG ACGACGGGAG GGCTTACTAT GTTATGGACT TCTGGGGTCA AGGAACCTCA 360
   GTCACCGTCT CCTCA 375
(2) INFORMATION FOR SEQ ID NO: 9:
   (i) SEQUENCE CHARACTERISTICS:
      (A) LENGTH: 339 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 9:
   GAGCTCCAGA TGACCCAGTC TCCATCATCT CTGGCTGCGT CTGCAGGAGA AAAGGTCACT 60
   ATGAGCTGTA AGTCCAGTCA AAGTGTTTTA TACAGTTCAA ATCAGAAGAA CTACTTGGCC 120
   TGGTACCAGC AGAAACCAGG GCAGTCTCCT AAACTGCTGA TCTACTGGGC ATCCACTAGG 180
   GAATCTGGTG TCCCTGATCG CTTCACAGGC AGTGGATCTG GGACACATTT TACTCTGACC 240
   GTCAGCAGTG TGCAAGCTGA AGACCTGGCA GTTTATTACT GTCATCAATA CCTCTACTCG 300
   TGGACGTTCG GTGGAGGCAC CAACCTGGAA ATCAAACGG 339
(2) INFORMATION FOR SEQ ID NO: 10:
   (i) SEQUENCE CHARACTERISTICS:
      (A) LENGTH: 759 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 10:
   CAGGTCCAAC TGCAGCAGTC TGGACCTGAG CTGGAGAAGC CTGGCGCTTC AGTGAAGATA    60
   TCCTGCAAGG CTTCTGGTTA CTCATTCACT GACTACAACA TGAACTGGGT GAAGCAGAGC   120
   AATGGAAAGA GCCTTGAGTG GATTGGAAAT ATTGATCCTT ACTATGGTGG TACTAGTTAC   180
   AATCAGAAGT TCAAGGGCAA GGCCACATTG ACTGTAGACA AATCCTCCAG CACAGCCTAC   240
   ATGCAGCTCA ACAGCCTGAC ATCTGAAGAC TCTGCAGTCT ATTTCTGTGC AAGATGGGAC   300
   TATAGGTACG ACGACGGGAG GGCTTACTAT GTTATGGACT TCTGGGGCCA AGGGACCACG   360
   GTCACCGTCT CCTCAGGTGG AGGCGGTTCA GGCGGAGGTG GCTCTGGCGG TGGCGGATCG   420
   GACATCGAGC TCACCCAGTC TCCATCATCT CTGGCTGCGT CTGCAGGAGA AAAGGTCACT   480
   ATGAGCTGTA AGTCCAGTCA AAGTGTTTTA TACAGTTCAA ATCAGAAGAA CTACTTGGCC   540
   TGGTACCAGC AGAAACCAGG GCAGTCTCCT AAACTGCTGA TCTACTGGGC ATCCACTAGG   600
   GAATCTGGTG TCCCTGATCG CTTCACAGGC AGTGGATCTG GGACACATTT TACTCTGACC   660
   GTCAGCAGTG TGCAAGCTGA AGACCTGGCA GTTTATTACT GTCATCAATA CCTCTACTCG   720
   TGGACGTTCG GTGGAGGCAC CAACCTGGAA ATCAAACGG                          759
(2) INFORMATION FOR SEQ ID NO: 11:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 11:
   TCTCACAGTG CACAGGTGCA GCTGCAGGAG TCTGG    35
(2) INFORMATION FOR SEQ ID NO: 12:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 12:
   CGTGAGAACA TATGGCGCGC CTTATTACCG TTTGATTTCC AGGTTGGTGC C 51
(2) INFORMATION FOR SEQ ID NO: 13:
   (i) SEQUENCE CHARACTERISTICS:
      (A) LENGTH: 764 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 13:
   GTGAAAAAAT TATTATTCGC AATTCCTTTA GTTGTTCCTT TCTATTCTCA CAGTGCACAG 60
   GTGCAGCTGC AGGAGTCTGG ACCTGGCCTG GTGAAACCCT CACAGAGCCT GTCCATCACA 120
   TGCACTGTCT CTGGGTTCTC ATTATCCAGA TATAGTGTAT ACTGGGTTCG CCAGCCTCCA 180
   GGAAAGGGTC TGGAGTGGCT GGGAATGATG TGGGGTGGTG GATCCACAGA CTATAATTCA 240
   GCTCTCAAAT CCAGACTGAC CATCAGCAAG GACACCTCGA AGAACCAGGT CTTCTTAAAA 300
   ATGAACAGTC TGCGAGCTGA GGACACAGCC ATGTACTACT GTGTCAGAAC CGATGGGGAC 360
   TACTGGGGCC AAGGGACCAC GGTCACCGTC TCCTCAGGTG GAGGCGGTTC AGACATTGAG 420
   CTCACCCAGT CTCCATCATC TCTGGCTGCG TCTGCAGGAG AAAAGGTCAC TATGAGCTGT 480
   AAGTCCAGTC AAAGTGTTTT ATACAGTTCA AATCAGAAGA ACTACTTGGC CTGGTACCAG 540
   CAGAAACCAG GGCAGTCTCC TAAACTGCTG ATCTACTGGG CATCCACTAG GGAATCTGGT 600
   GTCCCTGATC GCTTCACAGG CAGTGGATCT GGGACACATT TTACTCTGAC CGTCAGCAGT 660
   GTGCAAGCTG AAGACCTGGC AGTTTATTAC TGTCATCAAT ACCTCTACTC GTGGACGTTC 720
   GGTGGAGGCA CCAACCTGGA AATCAAACGG TAATAAGGCG CGCC 764
(2) INFORMATION FOR SEQ ID NO: 14:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 14:
   GCGCGAATTC ATGGACATGA GGGTCCCCGC 30
(2) INFORMATION FOR SEQ ID NO: 15:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 15:
   AGATTTGGGC TCAACTTTCT TGTCCAC 27
(2) INFORMATION FOR SEQ ID NO: 16:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 16:
   GTGGACAAGA AAGTTGAGCC CAAATCT 27
(2) INFORMATION FOR SEQ ID NO: 17:
   (i) SEQUENCE CHARACTERISTICS:
      (A) LENGTH: 82 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 17:
   GCGCGAATTC TTAAGCGGCC GCAGATCCGC CGCCACCCGA CCCACCTCCG CCCGAGCCAC 60
   CGCCACCTTT ACCCGGAGAC AG 82
(2) INFORMATION FOR SEQ ID NO: 18:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 18:
   GCGCGCGGCC GCAATGCACG TGGCCCAGCT T 31
(2) INFORMATION FOR SEQ ID NO: 19:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 19:
   GCGCGCGGCC GCCTAGTCAG AATCTGGGCA CGGTTC 36
(2) INFORMATION FOR SEQ ID NO: 20:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 20:
   GCGGCGGATC CATGGACATG AGGGTCCCCG C 31
(2) INFORMATION FOR SEQ ID NO: 21:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 21:
   GCGCGGATCC CTAACACTCT CCCCTGTTGA AGC 33
(2) INFORMATION FOR SEQ ID NO: 22:
   (i) SEQUENCE CHARACTERISTICS:
      (A) LENGTH: 876 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 22:
   ATGAAATACC TATTGCCTAC GGCAGCCGCT GGATTGTTAT TACTCGCGGC CCAGCCGGCC 60
   ATGGCCCAGG TCCAACTGCA GCAGTCTGGA CCTGAGCTGG AGAAGCCTGG CGCTTCAGTG 120
   AAGATATCCT GCAAGGCTTC TGGTTACTCA TTCACTGACT ACAACATGAA CTGGGTGAAG 180
   CAGAGCAATG GAAAGAGCCT TGAGTGGATT GGAAATATTG ATCCTTACTA TGGTGGTACT 240
   AGTTACAATC AGAAGTTCAA GGGCAAGGCC ACATTGACTG TAGACAAATC CTCCAGCACA 300
   GCCTACATGC AGCTCAACAG CCTGACATCT GAAGACTCTG CAGTCTATTT CTGTGCAAGA 360
   TGGGACTATA GGTACGACGA CGGGAGGGCT TACTATGTTA TGGACTTCTG GGGCCAAGGG 420
   ACCACGGTCA CCGTCTCCTC AGGCGGTGGC GGATCGGACA TTGAGCTCAC TCAGTCTCCA 480
   CTCTCCCTGC CTGTCAGTCT TGGAGATCGA GCCTCCATCT CTTGCAGATC TAGTCAGAGC 540
   CTTGTAAACA GTAATGGAAA CACCTATTTA CATTGGTACC TGCAGAAGCC AGGCCAGTCT 600
   CCAAAGCTCC TGATCTACAA AGTTTCCAAC CGATTTTCTG GGGTCCCAGA CAGGTTCAGT 660
   GGCAGTGGAT CAGGGACAGA TTTCACACTC AAGATTAGCA GAGTGGAGGC TGAGGATGTG 720
   GGAGTTTATT ACTGCTCTCA AAGTACACAT GTTCCGTGGA CGTTCGGTGG AGGCACCAAG 780
   CTGGAAATAA AACGGGCGGC CGCAGAACAA AAACTCATCT CAGAAGAGGA TCTGAATGGG 840
   GCCGCACATC ACCATCATCA CCATTAATAA GAATCC 876

## Description

### Field of the Invention

This invention relates to methods of treating diseases in which the immune system is involved. In particular, this invention relates to methods of preventing T cell mediated immune responses such as transplant rejection, autoimmune diseases or situations where T cell dependent immune responses are generated against a therapeutic molecule or carrier such as gene therapy vehicles.

### Background of the invention

### Activation of T cells:

Activation of T cells is known to require multiple interactions with antigen presenting cells (APC). The TcR-CD3 complex has two functions in antigen-induced activation: a recognition function in which a specific antigen is recognised in the context of the appropriate MHC molecule, and a signalling function in which the recognition event is transmitted across the plasma membrane. However, to induce proliferation and maturation into effector cells, T cells need a second signal in addition to the one mediated by the TcR-CD3 complex. This costimulatory signal is normally provided by the cell surface of APC. Intercellular signalling after TcR/MHC-peptide interaction in the absence of the costimulatory signal results in T cell inactivation, which is known as T cell anergy or T cell unresponsiveness. Several accessory molecules present on the cell surface of T cells with known ligands on the APC have been implicated in providing the costimulatory signal in T cell activation.

### The B7-CD28 costimulatory pathway:

The best candidate costimulatory signal leading to full T cell activation, is generated by interaction of CD28 on the T cells with the B7 costimulatory molecules on APC. *In vitro* studies have demonstrated that signaling via the CD28 costimulatory pathway can prevent the induction of anergy.

To date, two members of the B7 family have been molecularly cloned and functionally characterized: B7.1 (CD80), originally named B7/BB1 and a second B7 molecule, named B70 or B7.2 (CD86). CD80 is a monomeric transmembrane glycoprotein with an apparent molecular mass of 45-65 kDa and is, like CD28, a member of the immunoglobulin superfamily (Freeman et al., *J*. *Immunol*. 143:2714 (1989)). Initially it was reported that the expression of the CD80 molecule was restricted to activated B cells and monocytes stimulated with IFN-γ (Freedman et al., *Cell Immunol*. 137:429 (1991)). More recently, CD80 expression has also been found on cultured peripheral blood dendritic cells (Young et al., *J. Clin. Invest*. 90:229 (1992)) and on in vitro activated T cells (Azuma et al., *J. Exp*. *Med.* 177:845 (1993)). CD86 is a transmembrane glycoprotein with an apparent molecular mass of approximately 70 KDa and is also a member of the immunoglobulin superfamily (Freeman et al., *Science* 262:909 (1993)); Azuma et al., *Nature* 366:76 (1993)). The CD86 molecule seems to have a very similar distribution pattern to CD80, with the exception that induction of cell-surface expression seems to be faster and that it is present on freshly isolated monocytes.

It is clear from the literature that blocking CD80 exclusively only results in partial inhibition of T cell activation. Activation of T cells by alloantigen-expressing monocytes is predominantly dependent on CD86 costimulation. During an MLC with monocytes as stimulator cells, anti-CD86 Mab alone could strongly but not completely inhibit the proliferative response. However, only CTLA4-Ig (a soluble fusion protein consisting of the extra-cellular domain of human CTLA4 linked to human IgG [CH2 and CH3 domains], that can bind to both CD80 and CD86) or a combination of anti-CD80 plus anti-CD86 Mabs gave maximal inhibition.

### CD40-CD40L costimulatory pathway:

From the above reviewed literature data it appears that other costimulatory molecules can be of crucial importance for the activation of T cells when costimulation via CD80/CD86-CD28 is blocked. One such an alternative costimulatory pathway is regulated by the interaction between CD40 on the APC and CD40L on the T cells. The CD40 molecule belongs to the TNF receptor family of type I transmembrane proteins. The members of this gene family, which include: the two receptors for TNF; the low-affinity nerve growth factor receptor; the T cell activation antigen CD27; CD30 and; CD95, are characterized by sequence homology in their cysteine-rich extracellular domains (Armitage et al., *Current Opinion in Immunology* 6:407 (1994)). Interestingly, the known ligands for the members of the TNF receptor family are very homologous as well, forming another gene family named the TNF/CD40L gene family. Although TNF-α is a soluble cytokine, it is initially synthesized as a membrane associated molecule. Most of the members of the TNF/CD40L receptor family are type II transmembrane proteins.

CD40 is best known for its function in B cell activation. The molecule is constitutively expressed on all B cells. CD40L-CD40 interaction can stimulate the proliferation of purified B cells and, in combination with cytokines, mediate immunoglobulin production. Recent studies indicate that the distribution of the CD40 molecule is not so restricted as was originally postulated. Freshly isolated human monocytes express low levels of the CD40 molecule, which can be up-regulated by culturing in the presence of IFN-γ (Alderson et al., *J*. *Exp*. *Med.* 178:669 (1993)). Stimulation of monocytes via CD40 results in the secretion of pro-inflammatory cytokines such as IL-1 and TNF-α, toxic free radical intermediates such as nitric oxide, and up-regulation of the B7 costimulatory molecules. Human dendritic cells (DC) isolated from peripheral blood can also express the CD40 molecule (Caux et al., *J*. *Exp*. *Med.* 180:263 (1994)). Ligation of CD40 on DC results in enhanced survival of these cells when cultured in vitro. In addition, like with monocytes, stimulation of DC results in secretion of pro-inflammatory cytokines such as IL-1 and TNF-_α and up-regulation of the CD80/86 co-stimulatory molecules.

All the above described observations clearly indicate that the CD40L molecule on activated T cells is an important effector molecule that mediates stimulatory effects via ligation of CD40 expressed on a variety of cell types involved in the immunoinflammatory response. However, there is also experimental evidence that the CD40L molecule can receive signals that result in the costimulation of the T cell itself. Using mouse P815 cells that can present anti-CD3 monoclonal antibody to human T cells via binding to Fc-receptors on its cell surface, it was demonstrated that CD40 transfected P815 cells could substantially induce proliferation and CTL activity of small resting human T cells (Cayabyab et al., *J*. *Immunol*. 152:1523 (1994)). This demonstrates that the CD40L-CD40 interactions is clearly bi-directional.

### Prolonged transplant survival after blocking CD80/CD86-CD28:

Several recent *in vivo* models have shown that induction of prolonged graft acceptance is possible by interruption of the CD80/CD86-CD28 pathway. Treatment with CTLA-4 immediately after xenogeneic human pancreatic islet transplantation in mice resulted in long-term graft survival (Lenschow et al., *Science* 257:789 (1992)). However, 90% of fully mismatched rat cardiac allografts were rejected in rats treated intraperitoneally (IP) with CTLA-4Ig during 7 days (Turka et al., *Proc*. *Natl*. *Acad*. *Sci*. *USA* 89:11102 (1992)). CTLA-4Ig given intravenously (IV) at time of transplantation and then IP every other day on days 2 through 12, prolonged cardiac allograft survival in mice, but failed to prolong the survival of primary skin grafts (Pearson et al., *Transplantation* 57: 1701 (1994)). Blockage of the CD28-pathway with CTLA-4Ig resulted in significant prolongation of small bowel transplant survival in rats compared to controls, although all grafts were rejected after 15 days (Pescovitz et al., *Transplant Proc.* 26:1618 (1994)). Finally, treatment with CTLA-4Ig could reduce lethal murine GVHD in recipients of fully allogeneic bone marrow and significantly prolonged survival rates with up to 63% of mice surviving greater than 3 months post-transplantation (Blazar et al., *Blood* 83:3815 (1994)). The failure of CTLA-4Ig alone to induce anergy *in vitro* and *in vivo*, can most likely be explained by a persistent IL-2 production, induced by TCR triggering in combination with signaling from other accessory molecules on APC.

### Prolonged transplant survival after blocking CD40-CD40L:

Recent work has also demonstrated that blocking the CD40-CD40L pathway is strongly immunosuppressive in transplantation models. Combined treatment with allogeneic small lymphocytes or T cell-depleted small lymphocytes plus an antibody to mouse CD40L permitted indefinite pancreatic islet allograft survival in 37 out of 40 recipients that differed in major and minor histocompatibility loci (Parker et al., *Proc*. *Nat*. *Acad*. *Sci*. *USA* 92:9560 (1995)). From these experiments it was concluded that the effective blocking of the CD40L-CD40 interaction most likely had resulted in preventing the induction of costimulatory molecules on the small resting lymphocytes by the allorcactive host T cells. In another recent study, it was demonstrated that administration of a blocking Mab to mouse CD40L at the time of transplantation markedly prolonged survival of fully disparate murine cardiac allografts in both naive and sensitized hosts. However, when anti-CD40L therapy was delayed until postoperative day 5, anti-CD40L failed to prolong graft survival. From this study, it was concluded that anti-CD40L therapy inhibited allograft rejection primarily by interfering with T cell help for effector functions.

### Long-term acceptance of allografts without signs of chronic rejection after blocking CD80/CD86-CD28 and CD40-CD40L:

Blocking either of the two major T cell costimulatory pathways, CD80/86-CD28 or CD40-CD40L, alone is not sufficient to permit indefinite engraftment of highly immunogenic allografts. However, it was recently shown that blocking the CD80/CD86-CD28 and CD40-CD40L pathways simultaneously effectively aborts T cell clonal expansion *in vitro* and *in vivo*, promotes long-term survival of fully allogeneic skin grafts, and inhibits the development of chronic vascular rejection of primarily vascularized cardiac allografts (Larsen *et al*., *Nature* 381:434 (1996)). In the vascularizcd murine cardiac allograft model, C3HJ recipients treated with CTLA4-Ig alone (Mean Survival Time (MST) 50d), anti-CD40L Mab alone (MST 70d), or the combination (MST >70d), all showed prolonged survival of BALB/c cardiac allografts compared with untreated controls (MST 12d). Interestingly, when examined histologically at day 58-62 post-transplantation, marked differences were apparent. Allografts from CTLA4-Ig-treated mice showed extensive lymphocytic infiltration, interstitial fibrosis, and severe coronary arterial intimal thickening and fibrosis, all clear signs of a chronic rejection process. Anti-CD40L-treated recipients had less lymphocytic infiltration and interstitial fibrosis, but also had coronary vasculopathy characteristic of chronic rejection. In contrast, the allografts from mice in which the CD80/86-CD28 and CD40-CD40L pathways were blocked simultaneously, the parenchyma and blood vessels were virtually indistinguishable from those found in normal BALB/c hearts.

### Problems posed in the present invention:

Current concepts on immunological tolerance hold that anergy is the result of intercellular signalling after TcR/MHC-peptide interaction, in the absence of a so-called co-stimulatory signal. As described above, both the CD80/CD86-CD28 costimulatory pathway and the CD40L-CD40 costimulatory pathway arc important for the activation of T cells and play a role in the prevention of anergy. Stimulation of T cells via the TcR/CD3 complex results in a low and transient expression of CD40L. It has previously been demonstrated that stimulation of T cells via TcR/CD3 and costimulation with CD80/CD86-CD28 results in a strong and prolonged expression of the CD40L molecule. Low levels of CD86 can be found constitutively on various APC populations. Furthermore, stimulation of APC via CD40 is one of the strongest signals to up-regulate CD80 and CD86. Likewise, low levels of CD40L are expressed on T cells after first encounter of antigen (TcR/CD3 activation), even without complete costimulation. This CD40L expression can not only receive a signal from the APC via CD40, but can also stimulate the APC the enhance the CD86 expression and most importantly up-regulate CD80 expression. At the same time, low levels of CD86 expression on professional APC can prevent the induction of T cell anergy and up-regulation of CD80 and CD86 expression strongly stimulates the T cells to secrete cytokines and to enhance CD40L expression. It is therefore concluded by the present inventors that these two interactions (CD40L-CD40 and CD80/CD86-CD28) are connected in the initiation and amplification of T cell mediated immune responses. This has significant implications for optimal suppression of the immune response, since it can be expected that only blocking the CD40L-CD40 interaction will not completely prevent activation of the T cells via CD80/CD86-CD28 and only blocking the CD80/CD86-CD28 interaction will not completely prevent the activation of the effector functions of the APC population. From the these findings it is concluded that for optimal immunosuppression, the CD80/CD86-CD28 and CD40L-CD40 interaction need to be blocked simultaneously.

It is however not obvious that this can be accomplished with one single pharmaceutical agent, since no such reagent is presently available. In the above described *in vivo* experiments a combination of a monoclonal antibody to mouse CD40L was used in combination with CTLA4-Ig to block both CD80 and CD86. However, the generation of a single pharmaceutical agent by combining the ligand binding domains of CTLA4-Ig and that of an anti-CD40L monoclonal antibody will result in the cross-linking of T cells and APC. This will undoubtedly result in the activation of the T cells, which is exactly the opposite of the desired effect. Furthermore, all of the above referenced publications clearly suggest that blocking both CD80 and CD86 results in a better immunosuppression than blocking each of them separately. Also, it has been demonstrated that only blocking both CD80 and CD86 can result in T cell anergy. It was therefore surprising to find that in combination with an antagonistic anti-CD40 monoclonal antibody, blocking only CD86 resulted in T cell anergy despite the fact that CD80 was not blocked. This had led to the present invention that consists of pharmaceutical molecules that have the capacity to bind and block both the CD40 and the CD86 molecules.

### Summary of the Invention

The invention is based on the discovery that a molecular combination of an antagonistic molecule binding to CD40 and an antagonistic molecule binding to CD86, both expressed at low levels on professional APC, can potently inhibit the activation of T cells and even result in T cell anergy. Accordingly, this combination can be used to prevent or treat diseases in which the activation of T cells is involved.

Accordingly, it is a primary object of this invention to provide a pharmaceutical composition comprising of a therapeutically effective amount of a single soluble molecule capable of binding to the human CD40 and CD86 antigens located on the surface of antigen presenting cells.

It is another object of this invention to provide a method for treating T cell mediated diseases such as transplant rejection, multiple sclerosis, psoriasis, rheumatoid arthritis and systemic lupus erythematosus in a patient, the method comprising administering to a patient in need of such treatment a therapeutically effective amount of single soluble ligand capable of binding to the human CD40 and CD86 antigens located on the surface of antigen presenting cells, in a pharmaceutically acceptable excipient.

The preferred pharmaceutical composition to be used in this treatment is a single protein encompassing a combination of a therapeutically active antagonistic monoclonal antibody to CD40 or fragments thereof and a therapeutically active antagonistic CD86 ligand such as a monoclonal antibody to CD86, the CTLA4-Ig molecule or fragments thereof.

A more preferred pharmaceutical composition is a single protein encompassing a combination of a therapeutically active antagonistic monoclonal antibody to CD40 or fragments thereof and the therapeutically active antagonistic anti-CD86 monoclonal antibody Fun-1 (Nozawa et al., *J. Pathol*. 169:309 (1993)) or a therapeutically active fragment thereof.

### Detailed description of the invention

The invention is defined in the appending claims.

As used herein, a molecule capable of binding to an antigen typically denotes, but is not restricted to, an antibody. Other molecules having specific binding capacities such as ligands are also included.

As used herein, the term "antibody" refers to polyclonal antibodies, monoclonal antibodies, chimeric antibodies, humanized antibodies, single-chain antibodies, and fragments thereof such as F_{ab}, F_{(ab')2}, Fᵥ, and other fragments which retain the antigen binding function of the parent antibody.

As used herein, the term "monoclonal antibody" refers to an antibody composition having a homogeneous antibody population. The term is not limited regarding the species or source of the antibody,nor is it intended to be limited by the manner in which it is made. The term encompasses whole immunoglobulins as well as fragments such as Fab, F(ab')2, Fv, and others which retain the antigen binding function of the antibody. Monoclonal antibodies of any mammalian species can be used in this invention.

As used herein, the term"chimeric antibodies" means that the constant regions of an immunoglobulin are derived from human immunoglobulin sequences.

As used herein, the term "humanized antibodies" means that at least a portion of the framework regions of an immunoglobulin are derived from human immunoglobulin sequences.

As used herein, the term "single chain antibodies or ScFv" refers to antibodies prepared by determining the binding domains (both heavy and light chains) of a binding antibody, and supplying a linking moiety which permits preservation of the binding function. This forms, in essence, a radically abbreviated antibody, having only that part of the variable domain necessary for binding to the antigen. Determination and construction of single chain antibodies are described in U.S. Patent 4,946,778 to Ladner et al.

As used herein, the term "bi-specific diabody" refers to a single molecule in which two single chain antibody fragments with two different binding specificities fold together to form one molecule with the combined binding specificity of the two single chain antibody fragments.

As used herein, the term "bi-specific triabody" refers to a single molecule in which two single chain antibody fragments of one specificity fold together with one single chain antibody fragment of another specificity, to form one molecule with the combined binding specificity of the two different single chain antibody fragments.

As used herein, the term "tri-specific triabody" refers to a single molecule in which three different single chain antibody fragments with different binding specificities fold together to form one molecule with the combined binding specificity of the three single chain antibody fragments.

As used herein, the terms "CD80", "CD86", "CD40" and "CD40L" refer to human surface molecules as extensively reviewed above. For immunization purposes CD80, CD86, CD40 and CD40L antigen may be prepared by any technique known in the art. Antibodies to human CD80, CD86, CD40 and CD40L are known in the art. The present invention contemplates a new use for such antibodies as detailed above.

As used herein, the terms "CTLA4-Ig" refer to a soluble fusion protein consisting of the extra-cellular domain of human CTLA4 linked to human IgG [CH2 and CH3 domains] (Linsley et al., *J*. *Exp*. *Med*. 174: 561 (1991)).

As used in the present invention, the terms "conjugate" and "fusion protein" refers to the result from any type of coupling between two soluble ligands capable of binding to the human CD40 andCD86 antigens located on the surface of antigen presenting cells, which can be brought about by any technique known in the art. Such techniques include chemical coupling by any of a wide variety of known chemical procedures, or the fusion of two soluble molecules into a single chain molecule by recombinant DNA technology.

As used herein, the term "antagonistic" refers to the capacity of a soluble ligand to bind to an antigen located on the membrane of a target cell, where said binding prevents the binding of the natural ligand for said antigen resulting in the prevention of intracellular signal transduction leading to the activation of said target cell by said natural ligand. Antagonistic molecular antibodies that can bind to the human CD40 molecule located on the cell surface are described by Alderson et al, *J. Exp*. *Med*. **198** (1993), 669.

As used herein, the term "non-simulatory antagonistic" refers to the capacity of a soluble ligand to bind to an antigen located on the cell surface, where said binding prevents the binding of the natural ligand for said antigen resulting in the prevention of intracellular signal transduction leading to the activation of said target cell induced by said natural ligand and furthermore where said binding by itself does not result in intracellular signal transduction leading to the activation of said cell. Non-stimulatory aantagonistic monoclonal antibodies that can bind to the human CD40 molecule located on the cell surface are described in WO 94/01547.

As used herein, the term "cross-linking" refers to the physical interaction of two cells as a result of binding of a ligand or ligands expressed on these cells, where the said binding results in intracellular signal transduction leading to the activation of said cell.

The pharmaceutical compositions of this invention are administered at a concentration that is therapeutically effective to prevent allograft rejection, to prevent xenograft rejection, to treat autoimmune diseases, or to prevent the induction of a T cell dependent immune response to a therapeutic molecule or a therapeutic carrier such as gene therapy vectors. To accomplish this goal, the active molecules may be formulated using a variety of acceptable excipients known in the art. Typically, the pharmaceutical composition is administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Before administration to patients, formulants may be added to the pharmaceutical composition. A liquid formulation is preferred. For example, these formulants may include oils, polymers, vitamins, carbohydrates, amino acids, salts, buffers, albumin, surfactants, or bulking agents. Preferably carbohydrates include sugar or sugar alcohols such as mono, di, or polysaccharides, or water soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxyethyl starch and carboxymethylcellulose, or mixtures thereof. Sucrose is most preferred. "Sugar alcohol" is defined as a C₄ to C₈ hydrocarbon having an -OH group and includes galactitol, inositol, mannitol, xylitol, sorbitol, glycerol, and arabitol. Mannitol is most preferred. These sugars or sugar alcohols mentioned above may be used individually or in combination. There is no fixed limit to amount used as long as the sugar or sugar alcohol is soluble in the aqueous preparation. Preferably, the sugar or sugar alcohol concentration is between 1.0 w/v% and 7.0 w/v%, more preferable between 2.0 and 6.0 w/v%. Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. It is also preferred to use a buffer in the composition to minimize pH changes in the solution before lyophilization or after reconstitution. Most any physiological buffer may be used, but citrate, phosphate, succinate, and glutamate buffers or mixtures thereof are preferred. Most preferred is a citrate buffer. Preferably, the concentration is from 0.01 to 0.3 molar. Surfactants that can be added to the formulation are shown in EP-A 270,799 and 268,110.

Additionally, the binding molecules can be chemically modified by covalent conjugation to a polymer to increase their circulating half-life, for example. Preferred polymers, and methods to attach them to peptides, are shown in U.S. Patents 4,766,106, 4,179,337, 4,495,285, and 4,609,546 which are all hereby incorporated by reference in their entireties. Preferred polymers are polyoxyethylated polyols and polyethylene glycol (PEG). PEG is soluble in water at room temperature and has the general formula:R(O-CH₂-CH₂)ₙO-R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1000 and 40,000, more preferably between 2000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. Water soluble polyoxyethylated polyols are also useful in the present invention. They include polyoxyethylated sorbitol, polyoxyethylated glucose, polyoxyethylated glycerol (POG), etc. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG. The structure for POG is shown in Knauf et al. (*J. Bio. Chem.* 263:15064, 1988)), and a discussion of POG/IL-2 conjugates is found in U.S. Patent 4,766,106, both of which are hereby incorporated by reference in their entireties.

As stated above, the pharmaceutical compositions of this invention are used to prevent allograft rejection, to prevent xenograft rejection, or to treat autoimmune diseases. The preferred route of administration is parentally. In parental administration, the compositions of this invention will be formulated in a unit dosage injectable form such as a solution, suspension or emulsion, in association with a pharmaceutically acceptable parental vehicle. Such vehicles are inherently nontoxic and nontherapeutic. Examples of such vehicles are saline, Ringer's solution, dextrose solution, and Hanks' solution. Nonaqueous vehicles such as fixed oils and ethyl oleate may also be used. A preferred vehicle is 5% dextrose in saline. The vehicle may contain minor amounts of additives such as substances that enhance isotonicity and chemical stability, including buffers and preservatives.

The dosage and mode of administration will depend on the individual. Generally, the compositions are administered so thatthey are given at a dose between 0.1 mg/kg and 10 mg/kg. Preferably, it is given as a bolus dose. Continuous infusion may also be used. If so, the pharmaceutical compositions may be infused at a dose between 0.05 and 1 mg/kg/hour.

The present invention will now be illustrated by reference to the following examples which set forth particularly advantageous embodiments. However, it should be noted that these embodiments are illustrative and are not to be construed as restricting the invention in any way.

### Brief Description of the figures

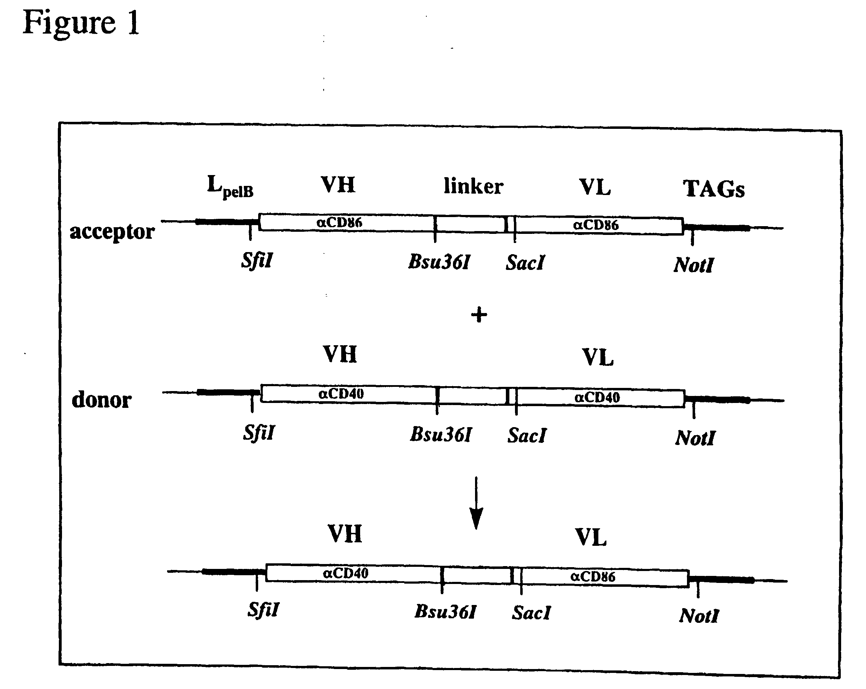

Figure 1 shows a schematic drawing of the first step in the diabody construction, exchange of the V-regions.

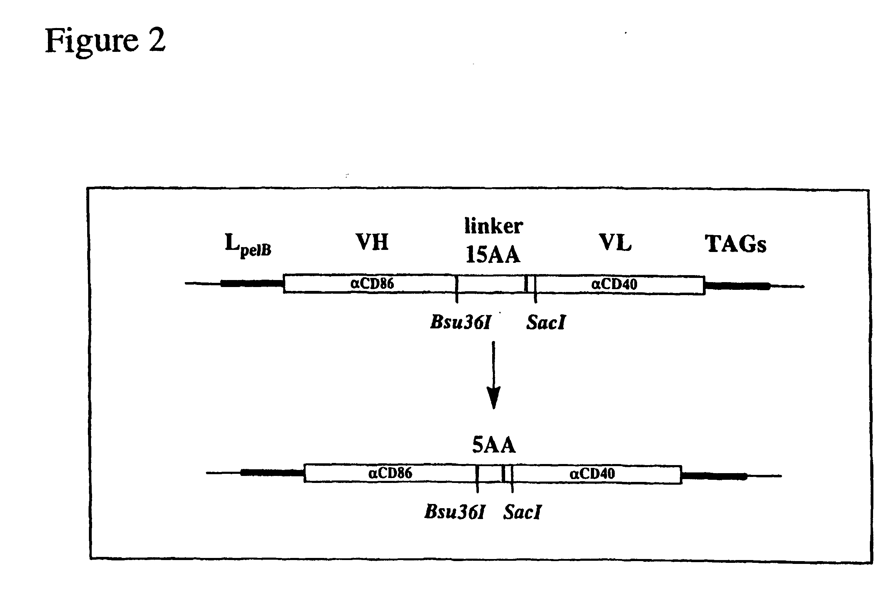

Figure 2 shows a schematic drawing of the exchange of the linker, the second step in the diabody construction.

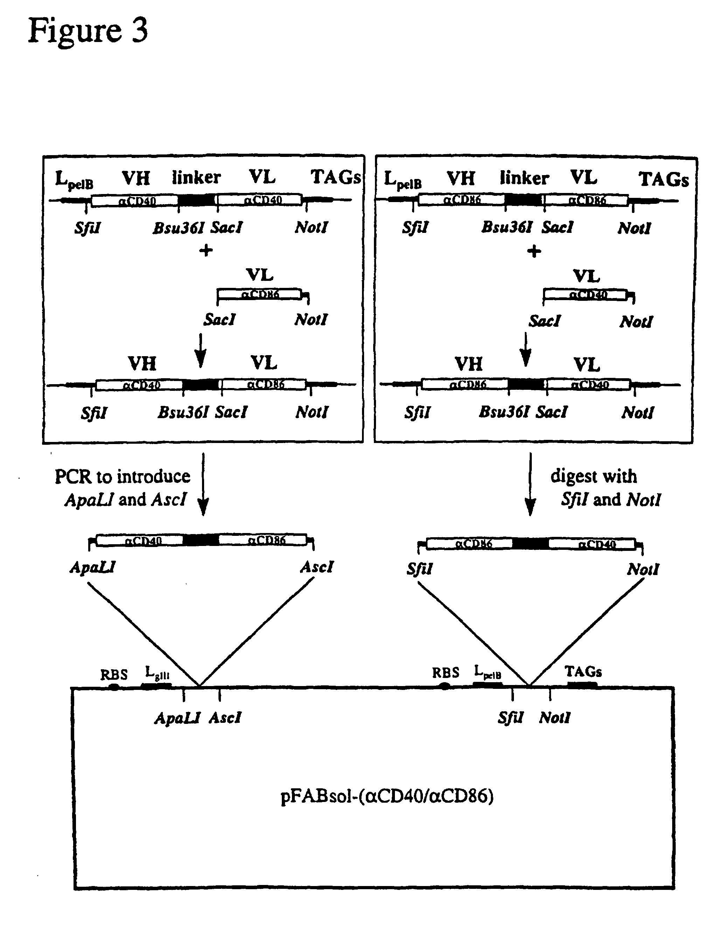

Figure 3 shows a schematic drawing of the strategy applied for cloning of the anti-CD40/anti-CD86 diabody.

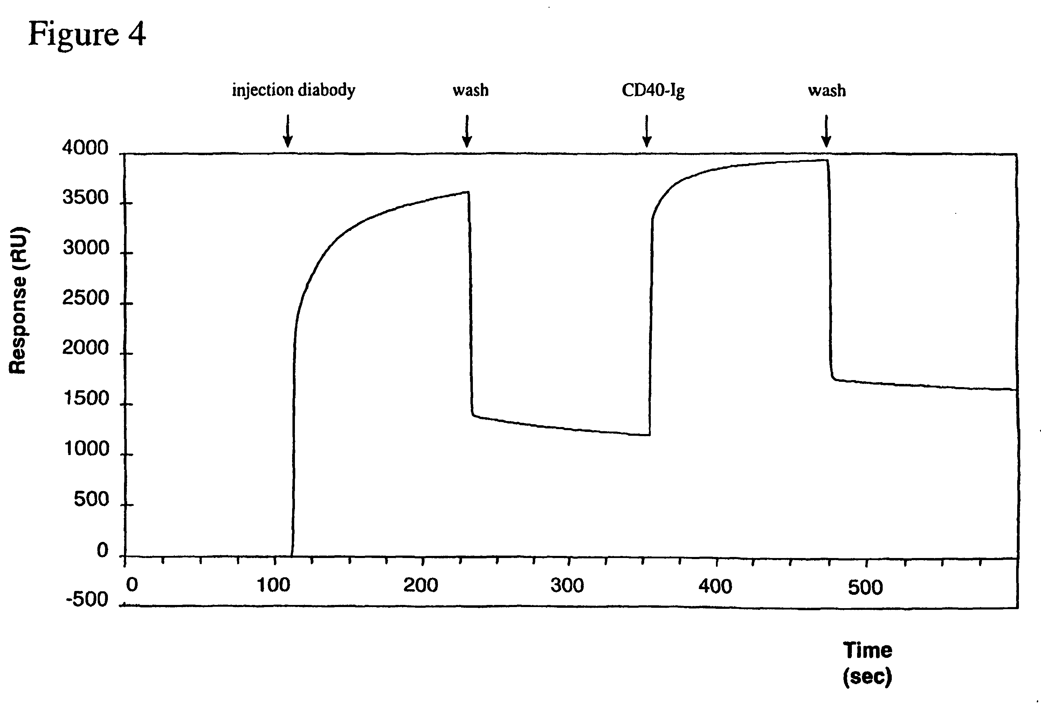

Figure 4 shows a BIAcore sensogram showing the capability of the diabody to bind the CD86-Ig and CD40-Ig antigen simultaneously. From the periplasmic fraction, injected at t=110 (sec) on a surface containing 6537 Response Units (RU) CD86-Ig, 1300 RU diabody was captured at t=230 (sec), as can be seen by the difference in response after the first wash and the normalized signal before injection. At t=360 (sec) CD40-Ig was injected during 120 sec. The diabody, arrested with one binding domain to the CD86-Ig, reacted with the other domain to CD40-Ig yielding 550 RU captured antigen (difference in signal between the second wash at t=480 (sec) and before the second injection at t=360 (sec)).

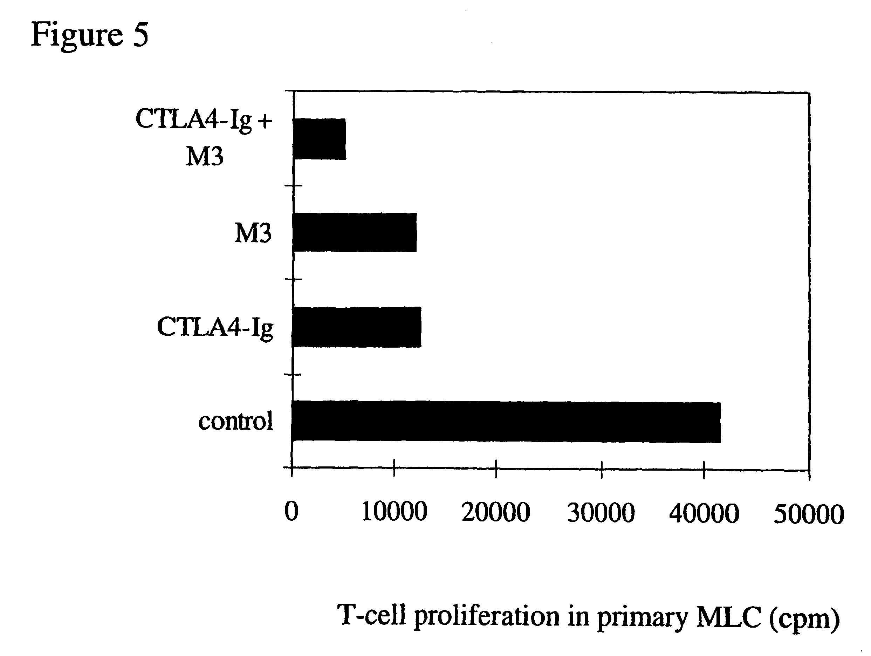

Figure 5 shows the results of a T cell activation experiments in which T cells are stimulated with allogeneic monocytes. T cell activation is partially inhibited by blocking CD80 and CD86 with CTLA4-Ig or an antagonistic anti-CD40 monoclonal antibody M3 alone, but almost completely blocked when CTLA4-Ig and antagonistic anti-CD40 monoclonal antibody M3 are combined.

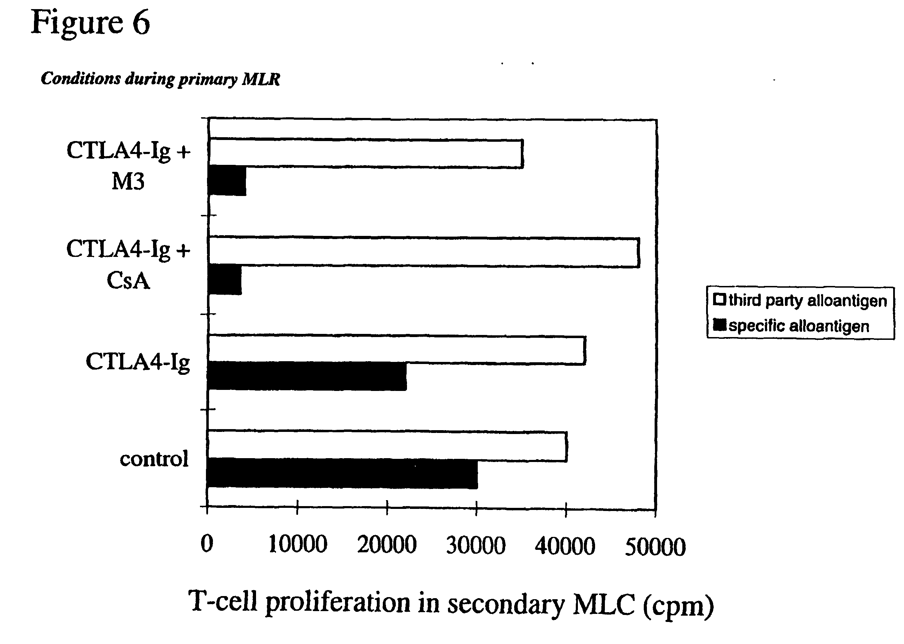

Figure 6 shows the results of a T cell restimulation experiments in which T cells are stimulated with allogeneic monocytes in the presence of blocking agents during primary stimulation and analysed for proliferative capacity during restimulation in the absence of blocking agents. The presence of CTLA4-Ig alone only results in a slight alloantigen-specific hypo-responsiveness, whereas the combination of CTLA4-Ig with CsA or the antagonistic anti-CD40 monoclonal antibody M3 results in T cell unresponsiveness. T cell responses to the control third-party alloantigen-expressing monocutes is not affected. Closed bars are the T cell responses to the alloantigen used in the primary culture, open bars represent T cell responses to third-party alloantigen-expressing monocytes.

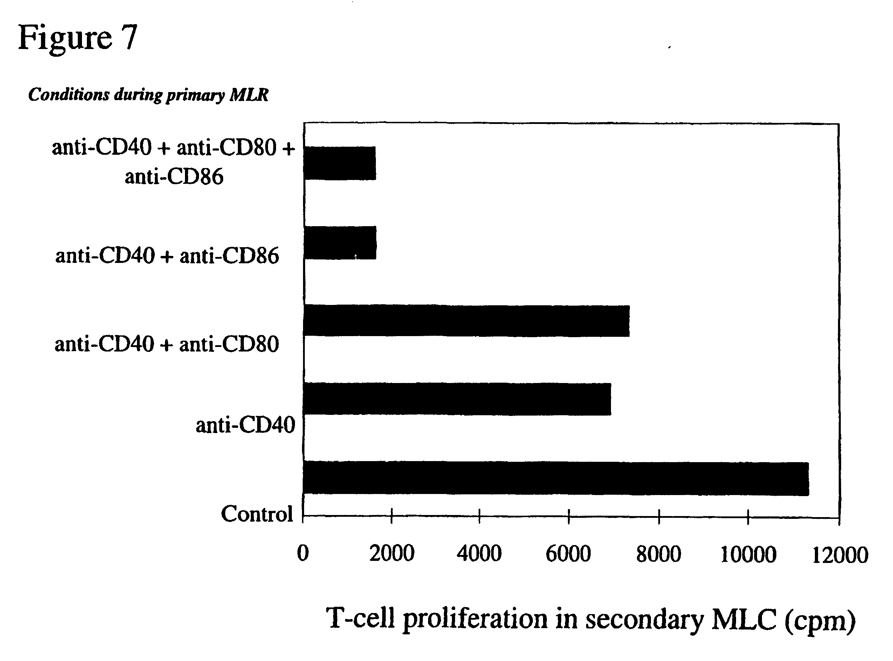

Figure 7 shows the results of a T cell restimulation experiments in which T cells are stimulated with allogeneic PBMC in the presence of blocking agents during primary stimulation and analysed for proliferative capacity during restimulation in the absence of blocking agents. The presence of antagonistic monoclonal antibody to CD40 and antagonistic monoclonal antibody to CD86, with or without antagonistic monoclonal antibody to CD80 results in alloantigen-specific T cell unresponsiveness. The presence of antagonistic monoclonal antibody to CD40 alone or in combination with antagonistic monoclonal antibody to CD80 only results in partial inactivation of the T cells.

### Examples

### Materials and Methods:

### CD40 and CD86 extracellular domain Ig fusion proteins

In several experiments fusion proteins of lymphocyte cell surface receptors and the Fc-region of human IgG were used. These extracellular domain (ED) fusion proteins (ED-Ig fusion proteins) have been generated by fusion of nucleic acid sequence encoding the extracellular domain of the cell surface receptors generated by PCR amplification based on published cDNA sequences to the CH1/hinge-CH3 region (Fc) of human IgG1 based on the sequence by Ellison et al. (*NAR* 10:4071 (1982)). ED-Ig fusion proteins were expressed in Sf9 insect cells and were used as conditioned medium or after purification by affinity chromatography using protein A.

### Cell lines and culture conditions

The B-cell line JY was cultured in T75 culture flasks routinely (Costar, Cambridge, MA, USA) in Iscove's modified Dulbecco's medium (IMDM) to which 50 µg/ml gentamycin and 10% foetal calf serum was added (FCS) (Hyclone, Logan, Utah USA). The cells were cultured in a humidified incubator at 37ºC and 5% CO₂. Every week the cells were split (1/20 to 1/100). To store the cell line, ampoules were made containing 5-10 x 10⁶ cells/ml Hank's balanced salt solution HBSS supplemented with 20% FCS and 10% DMSO and stored in the liquid nitrogen.

### Lymphocyte isolation and stimulation

Peripheral blood mononuclear cells (PBMC) were isolated from heparinized blood from healthy donors by Ficoll-Hypaque density centrifugation and re-suspended in complete medium consisting of RPMI 1640 (Gibco, Paisley, UK) supplemented with 2 mM L-Glutamine, streptomycin (100 mg/ml), penicillin (100 U/ml) and 5% heat-inactivated autologous plasma. Enriched monocyte preparations were prepared by resetting of PBMC with AET-treated sheep red blood cells and removal of E-rosetting cells on Ficoll-Hypaque density gradients, followed by cold aggregation of monocytes as essentially described by Zupo et al. (*Eur. J. Immunol*. 21:351 (1991)). T cells were further purified from the PBMC preparations by depletion of monocytes, B cells and NK cells using Lympho-Kwik T (One Lambda, Los Angeles, CA, USA) according to the manufacturers protocol.

For primary mixed lymphocyte cultures with enriched monocytes and purified T cells, 0.5-1 x 10⁶/ml purified T cells and 0.1-0.2 x 10⁶/ml monocytes were cultured in 200 ml complete culture medium in 96-well plates for 6 days in the presence or absence of blocking agents in concentrations ranging from 1-10 µg/ml. For the last 8h of the culture period, cells were pulsed with 1 mCi [3H]-thymidine (Amersham International, Amersham, UK). Cells were harvested on glass fiber filters by using a Skatron automatic cell harvester, and radioactivity on the paper was counted in a liquid scintillation counter. For re-stimulation experiments, the T cells (0.5-1 x 10⁶/ml) and monocytes from the same donor (0.1 to 0.2 x 10⁶/ml) were cultured for 6 days in 1 ml complete medium in 24-well plates in the presence or absence of blocking agents in concentrations ranging from 1-10 µg/ml and in the presence or absence of cyclosporin A (CsA) at a concentration of 400 ng/ml. After 6 days the remaining cells were collected and cultured for an additional 2 days, before re-stimulation for 3 days in the absence of blocking agents. T cell proliferation was determined as described above for primary mixed lymphocyte cultures.

Mixed lymphocyte cultures were also performed with non-separated PBMC. In these experiments, fresh PBMC (1 x 10⁶/ml) as responder cells and PBMC that were pre-activated with human IL-4 (20 µg/ml) and human GM-CSF (100 U/ml) (0.1 x 10⁶/ml) as stimulator cells were cultured for 6 days in 1ml complete culture medium in 24-well plates in the presence or absence of blocking agents in concentrations ranging from 1-10 µg/ml. After 6 days the remaining cells were collected and cultured for an additional 3 days, before the allo-reactive T cells were re-stimulated with the same PBMC that were pre-activated with human IL-4 (20 µg/ml) and human GM-CSF (100 U/ml) (0.1 x 10⁶/ml) as stimulator cells for 3 days in the absence of blocking agents. T cell proliferation was assayed by [3H]-thymidine incorporation as described above.

### Polymerase chain reaction

To amplify DNA fragments, polymerase chain reactions (PCR) were performed. A typical PCR reaction mix contained: 0-10 mM MgCl₂, 50 mM KCI, 10 mM Tris-HCl pH 9.0, 1.0% Triton X-100, 0.25 mM dNTP each, 25 pmol primer/100 µl reactions mix, 1-1000 ng DNA/100 µl reaction mix and 2.5 U Taq polymerase. Reactions were run using a Perkin Elmer thermocycler (Perkin Elmer Corp, Norwalk CT). A stantard PCR scheme consisted of one step for 2-5 min at 95°C to denature the DNA, followed by 20-40 cycles of 1 min at 95ºC, 1 min at 55ºC and 1-4 min at 72°C. After the final step an extension step was performed for 7 min at 72°C.

### Flow cytometric analysis (FACS)

Cells (0.1-0.2 x 10⁶/sample) were incubated for 20 min at 4ºC with the specific monoclonal antibody (0.1-1 mg/sampic). After washing with FACS buffer (PBS pH 7.4 1% BSA 0.1% NaN₃), the cells were incubated for another 20 min at 4ºC with goat anti-mouse antibodies conjugated to fluorescein isothiocyanate (FITC) or phycoerythin (PE). The cells were washed with FACS buffer and finally suspended in FACS buffer containing 0.5% paraformaldehyde and analysed with a FACScan flow cytometer (Becton Dickinson). The specific binding of the monoclonal antibodies is expressed as the mean fluorescent intensity in arbitrary units. A similar protocol was used to test the single chain antibody expressing phage particles. In this case detection was done by using an unconjugated sheep anti-M13 antibody (Pharmacia AB, Uppsala Sweden), followed after washing by incubation with donkey anti-sheep conjugated to FITC (Sigma Chemical Co. St. Louis, MO, USA). Likewise a similar protocol was used to demonstrate the biological activity of the diabody and triabody constructs. In these experiments detection was done by incubation of the cells with the diabody or triabody constructs followed by incubation with one of the ED-Ig fusion proteins, followed by incubation with an FITC-conjugated anti-human IgG antiserum.

### SDS-PAGE and Western blotting analysis

To analyse the expressed constructs SDS-PAGE and Western blot analysis was performed. Briefly, samples were boiled for 5 min in 0.8% sodium dodecyl sulfate (SDS) and 1 mM dithiothreitol (DTT). Subsequently the samples were run on a 15% SDS-polyacrylamide gel electrophoresis (SDS-PAGE; 2h 100V). After electrophoresis the gel was electroblotted to a nitrocellulose filter or stained with 0.1% coommassie blue in 10% methanol and 10% acetic acid. Electroblotting was done in 25 mM Tris-HCl, 192 mM glycine and 10% methanol pH 8.3 for 1h at 100V; 4°C. After blotting the nitrocellulose filter was blocked with 1% BSA in PBS-Tween (0.05%) for 1h at room temperature. Subsequently the blot was incubated o/n room temperature in PBS-Tween with a anti c-myc antibody for detection. After incubation with a second antibody (peroxidase labelled), the blots were stained by 4-chloro-naphtol.

### BIACore analysis

Analysis were performed on the Pharmacia Biosensor 2000. The CM chip was activated with 0.2M EDC/0.05M NHS during 5 min. Subsequently coupling of ligand was done in 10mM NaAc pH5.0 during 5 min (0.1 mg Ig construct/ml). This was followed by loading of the ScFv, Mab or dia/triabody constructs in various concentrations in PBS buffer. In case of the dia/triabody constructs the chip was washed followed by loading of the second Ig contruct. The sensor surface was regenerated with 0.1M NaOH.

### Example 1

### Generation of single chain antibody fragments expressing phage from monoclonal antibodies to human CD40 and CD86:

For the generation of a single chain antibody fragment (ScFv) of the anti-CD40 monoclonal antibody 5D12 both the VH and VL region were amplified by PCR, followed by a second assembly PCR to connect both regions. For this purpose 4 primers were designed (SEQ ID NO:1-4). SEQ ID NO:1 contains a HindIII and SfiI restriction site for cloning purposes followed by a degenerated sequence annealing to the 5' VH region of 5D12. SEQ ID NO:2 contains a degenerate sequence for the 3' part of the VH region followed by a sequence encoding a ((Gly)₄Ser)₃linker and the 5' part of the VL regions. SEQ ID NO:3 is a degenerated primer having homology with the 5' part of the VL region, while the last primer (SEQ ID NO:4) contains a NotI restriction site and anneals to the 3' part of the VL region. Briefly, these primers were used to separately PCR amplify the VH and VL regions of monoclonal antibody 5D12. As template for this PCR reaction we used a plasmid containing the VH or VL regions of 5D12 (VH: SEQ ID NO:5 and VL: SEQ ID NO:6). The cDNA obtained in this PCR step was gel purified and used in an assembly PCR resulting in the linkage of the V region through the (Gly₄Ser)₃ linker. Subsequently the obtained single chain 5D12 construct was digested with the restriction enzymes HindIII and NotI, followed by ligation in pGEM-13Zf (Promega Madison USA). The ligation was transformed in DH5α and plated on LB plates. By sequencing of several clones, a correct 5D12 ScFv clone was found (SEQ ID NO:7).

For the generation of ScFv's reactive with human CD86, we used the same primer set as for 5D12. All the steps in the generation of the ScFv of the anti-CD86 monoclonal antibody Fun-1 were performed as described above for the 5D12 ScFv reactive with human CD40. The V regions of Fun-1 (VH: SEQ ID NO:8; VL: SEQ ID NO:9) were used as template to obtain the anti-CD86 ScFv construct (SEQ ID NO:10).

### Example 2

### Construction of bi-specific diabody molecules capable of binding to human CD40 and human CD86:

Bi-specific bivalent molecules were generated by shortening the flexible linker sequence in the anti-CD40 ScFv and in the anti-CD86 ScFv, from fifteen residues to five (Gly₄Ser) and by cross-pairing the variable heavy and light chain domains from the two single chain Fv fragments with the different antigen recognition. The construction was performed in three steps. The light chain variable fragments were exchanged in the ScFv constructs from anti-CD86 (aCD86) and anti-CD40 (aCD40) by using restriction enzyme sites located in the 5' end (SacI at nucleotide numbers 7 to 12 of VL) and just outside the 3' part of the light chain variable gene (NotI) (see Figure 1). In the following step the 15-residue linker of the chimeric constructs VH-aCD86/15AA-linker/VL-aCD40 (coded 7.2/15/40) and VH-aCD40/15AA-linker/VL-aCD86 (coded 40/15/7.2) was replaced by the 5 residue linker (Gly₄Ser) by using sites located in the 3' part of VH (Bsu361 at nucleotide number 335 of the anti-CD40 VH or at 371 of the anti-CD86 VH to number +2 in the linker sequence) and the 5' part of VL end (SacI at nucleotide number 7 to 12 both VL's) (see Figure 2). Finally, both chimeric cassettes were combined in the vector pUC119-fabsol (a pUC119 derivative similar to pUC119His6mycXba (Low et al., *J. Mol*. *Biol.* 260:359 (1996)), but with all Apall-sites in the vector backbone deleted by *in vitro* mutagenesis) containing a bi-cistronic expression cassette. The subcloning was performed in two steps. First, the aCD86/5AA-linker/ aCD40-construct was cloned in pUC119-fabsol using the restriction sites SfiI and NotI. Subsequently the ScFv cassette of aCD40/5AA-linker/aCD86 was amplified with the following oligonucleotide primers: 5'-TCT CAC AGT GCA CAG GTG CAG CTG CAG GAG TCT GG-3' (SEQ ID NO:11) and 5'-CGT GAG AAC ATA TGG CGC GCC TTA TTA CCG TTT GAT TTC CAG GTT GGT GCC-3' (SEQ ID NO:12). These primers contain an ApaLI-and an AscI-site respectively (underlined). The amplified PCR-fragment was digested with ApaLI and AscI, and ligated in the pUC119-fabsol plasmid containing the aCD86/5AA-linker/aCD40-construct. A diabody-producing clone containing both ScFv cassettes was identified and used for expression of the recombinant diabody molecule (pUC119-fabsol-CD40/CD86) (5D12VH + FUNVL: SEQ ID NO:13; FUNVH + 5D12VL: SEQ ID NO:22).

The whole cloning strategy to obtain the bi-specific diabody molecule based on antagonistic anti-CD40 Mabs and antagonistic anti-CD86 Mab Fun-1, capable of binding to human CD40 and human CD86 molecule on antigen presenting cells, is summarized in Figure 3. The same procedure is also used for the generation of a bi-specific diabody molecule based on antagonistic anti-CD40 Mabs and antagonistic Mabs reactive with both CD80 and CD86, capable of binding to human CD40, human CD80 and human CD86 molecule on antigen presenting cells.

### Example 3

### Expression, isolation and characterization of bi-specific diabody molecules capable of binding to human CD40 and human CD86:

For the production of the bi-specific diabody molecule capable of binding to human CD40 and human CD86 the plasmid containing the aCD86/aCD40-bicistronic expression cassette described in Example 2 above was used. Fifty ml 2YT medium (100 µg/ml amp, 0.1% glucose) was inoculated (1 v/v %) with a saturated culture (16 hours grown at 30°C). After 5 hours growth at 30°C and with a culture having an optical density at 600 nm of 0.9, the diabody production was induced by adding IPTG to a concentration of 1 mM. Cultivation was continued for 4 hours at 20°C and the cells from 15 ml culture were pelleted (10 minutes at 1100*g at room temperature). Supernatant and cells were stored at -20°C until further processed. The remaining 35 ml culture was grown at 20°C for another 16 hours. Cells were pelleted as described above. Periplasmic fractions were prepared by resuspending the cells in 0.53 ml cold TES-buffer (20 mM Tris-HCl; 0.5 mM EDTA; 0.5 M sucrose pH 8.0). The mixture was incubated for 2 minutes on ice, subsequently 0.59 ml cold three fold diluted TES was added and the incubation was prolonged with 30 minutes. The spheroplasts were centrifuged for 15 minutes at 1100*g at 4°C and the supernatant containing the periplasmic proteins was collected. The pellet fraction was resuspended in 0.75 ml TES/MgSO₄ (TES-buffer; 15 mM MgSO₄) and incubated for 30 minutes on ice. Spheroplasts were pelleted for 15 minutes at 1100*g at 4°C and the supernatant added to the first supernatant fraction. The total periplasmic fraction was cleared again (15 minutes at 1100*g at 4°C) and dialyzed against PBS. All fractions were analyzed on PAGE and Western blot with the anti-c-myc antibody for detection. The highest concentration of ScFv was found in the periplasmic fraction prepared from the culture after 4 hours induction, and to some degree in the medium fraction of the culture induced for 20 hours. The functionality of the produced diabody was tested in BIAcore. Purified CD86-Ig was immobilized on the surface of a CM-chip, yielding 6500 RU (Response Units) of coupled protein. Injection of the periplasmic fraction for 120 sec with a flow rate of 10 µl/min resulted in the capture of approx. 1200 RU diabody. Subsequently CD40-Ig was injected under the same conditions as the diabody resulting in the binding of an additional 540 RU antigen. This experiment demonstrated the capability of the produced diabody molecule to bind CD40 and CD86 simultaneously (see Figure 4).

### Example 4

### Construction of bi-specific and tri-specific triabody molecules capable of binding to human CD40 and CD86 or human CD40, CD80 and CD86:

The construction of bi-specific and tri-specific triabody molecules is analogous to the scheme described above for the diabody, except that the linker has to be deleted (zero residue linker). This is accomplished by *in vitro* mutagenesis, using single stranded phagemid DNA and oligonucleotides encoding the mutation. Using ScFv constructs of antagonistic monoclonal antibodies to human CD40, CD80 and CD86 at least two gene constructs are possible:(i) VHαCD40/0/VLαCD80-VHαCD80/0/VLαCD86-VHαCD86/0/VLαCD40 and; (ii) VHαCD40/0/VLαCD86-VHαCD86/0/VLαCD80-VHαCD80/0/VLαCD40. All VH/VL-combinations arc made by exchanging VH- and VL-domains in the constructs such as described above for bi-specific diabodies having the 5 AA linkers, applying the strategy described above using SfiI and Bsu361 to exchange VH regions, and SacI and NotI to exchange VL regions. Subsequently, the three ScFv-cassettes are cloned in a single expression module encoding a tricistronic mRNA. This DNA will serve as template for an oligo-directed in vitro mutagenesis procedure, to delete the 5 residue linker in one up to three VH-VL-pairs. The various triabodies that are made, may differ in binding characteristics due to other orientations of the ScFv domains and in linker length. Only one of the three ScFv cassettes is provided with the previously mentioned tag sequences. In order to drive triabody formation as well as to maintain stability, disulfide bridges can be introduced by adding cysteine residues at the carboxyterminus or within the V-regions.

### Example 5

### Construction of a fusion molecule consisting of an antagonistic anti-CD40 monoclonal antibody linked by its C-terminal residue to the extracellular domain of human CTLA4 capable of binding to CD40, CD80 and CD86:

The conceptual therapeutic agent is a fusion protein combining the high affinity and specificity of CTLA4 for both CD80 and CD86 with an antagonistic anti-CD40 monoclonal antibody. This fusion molecule is produced in stable, active form as a complete anti-CD40 monoclonal antibody to which the extracellular domain of human CTLA4 (CTLA4ED) is C-terminal linked by a flexible linker. The construction of the anti-CD40 antibody attached by its Fc part to the extracellular domain of CTLA4 is done by the following PCR and cloning steps. The VH and CH1 regions of anti-CD40 together with a leader sequence are amplified using the oligonucleotides 5' GCG CGA ATT CAT GGA CAT GAG GGT CCC CGC 3' (SEQ ID NO:14) and5' AGA TTT GGG CTC AAC TTT CTT GTC CAC 3' (SEQ ID NO:15). This is followed by amplification of the CH2 and CH3 regions of human IgG using the oligonucleotides 5'GTG GAC AAG AAA GTT GAG CCC AAA TCT 3'(SEQ ID NO: 16) and 5'GCGC GAA TTC TTA AGC GGC CGC AGA TCC GCC GCC ACC CGA CCC ACC TCC GCC CGA GCC ACC GCC ACC TTT ACC CGG AGA CAG 3' (SEQ ID NO: 17). After removing of the primers a second PCR is done to assemble both PCR products to obtain a full-length 5D12 heavy chain. The obtained PCR product is gel purified and cloned in pCR Script using the Stratagene cloning kit. Briefly, the PCR product is incubated with plasmid together with T4 ligase and SrfI for 1h at room temperature, after which the entire sample is transformed in XI1Blue E. coli cells. The cells are plated on LB plates containing 100 µg ampiciline/ml, 20 µg IPTG/ ml and 20 µg Xgal/ml. After incubation o/n at 37°C putative white clones are analyzed for having an insert. Clones containing inserts are analyzed by cycle sequencing using M13 and M13 reverse primers. After confirming the correct sequence the anti-CD40 heavy chain is cloned using the EcoRI restriction site in the bicistronic baculovirus expression plasmid pAcUW51 (Pharmingen) after the p10 promoter. The in this way obtained construct already contains C-terminal a flexible (Gly₄Ser)₃ linker after which the CTLA4ED part was cloned. Therefore the CTLA4ED part is amplified with the oligonucleotides 5' GCGC GCG GCC GCA ATG CAC GTG GCC CAG CCT G 3' (SEQ ID NO: 18) and 5' GCGC GCG GCC GC CTA GTC AGA ATC TGG GCA CGG TTC 3' (SEQ ID NO: 19) by PCR, gel purified and cloned after the heavy chain of 5D12 using the NotI cloning site. After confirmation by sequence analysis of this step the light chain is cloned. This is done by using a plasmid which already contained the VL region of 5D12 attached to a human CL region. So using the oligonucleotides 5' GCGC GGATCC ATG GAC ATG AGG GTC CCC GC 3' (SEQ ID NO: 20) and 5'GCGC GGATCC CTA ACA CTC TCC CCT GTT GAA GC 3' (SEQ ID NO: 21) the light chain of 5D12 is amplified and cloned in the constructed pAcUW51 expression plasmid using the BamHI cloning site after the polyhedrin promoter. After DNA sequence analysis a correct clone is obtained. After this confirmation, the expression plasmid containing the 5D12-CTLA4ED construct is introduced into Sf9 insect cells along with the viral AcNPV wild-type DNA using the BaculoGold transfection system of Pharmingen. Recombinant virus is plaque-purified and the integrity of the expression cassette is checked by PCR and cycle sequencing. For protein production, insect cells are used. These cells can grow in suspension in serum-free medium, and are the best known secretors of heterologous proteins. The fusion protein is purified from serum-free conditioned medium by *S*. *aureus* protein A affinity chromatography (Harlow and Lane, 1988). Purity is checked by SDS-PAGE and by western blotting under reducing and non-reducing conditions to assess the extent of dimerization.

### Example 6

### Effects of blocking the CD40L-CD40 and/or CD80/86-CD28 pathways on the activation of T cells:

It has been demonstrated extensively that blocking the CD80/86 pathway results in an inhibition of activation of T cells (reviewed in Van Gool et al., *Res. in Immunol*. 146:183 (1995)). However, under a number circumstances does blocking of the CD80/86 interaction not result in complete prevention of T cell activation. This is exemplified by Figure 5, in which purified human T cells are stimulated with allogeneic monocytes as detailed above under materials and methods. Addition of CTLA4-Ig (blocking both CD80 and CD86) only resulted in a partial inhibition of the alloantigen-specific T cells. Surprisingly, addition of the antagonistic anti-CD40 monoclonal antibody M3 also resulted in a partial inhibition of T cell activation to the same extend as CTLA4-Ig. Even more surprisingly, the combination of the antagonistic anti-CD40 monoclonal antibody M3 with CTLA4-Ig resulted in nearly complete blockade of T cell activation.

It has also been demonstrated that a blockade of CD80/86 in combination with CsA results in antigen-specific T cell unresponsiveness (reviewed in Van Gool et al., *Res. in Immunol*. 146:183 (1995)). This has been demonstrated in mixed lymphocyte cultures, in which blocking agents were added during a primary stimulation with the alloantigen, followed by a short rest period and subsequent restimulation with the same alloantigen in the absence of blocking agents. Figure 6 indeed shows that addition of CTLA4-Ig plus CsA, but not CTLA4-Ig alone to purified human T cells that are stimulated with allogeneic monocytes results in alloantigen-specific T cell unresponsiveness (solid bars). The response to unrelated third party alloantigen-expressing monocytes in unchanged (open bars). Figure 6 also shows that the addition of CTLA4-Ig plus anti-CD40 monoclonal antibody M3 alone to purified human T cells that are stimulated with allogencic monocytes also results in alloantigen-specific T cell unresponsiveness (solid bars). Again, this unresponsiveness to the alloantigen of the first culture is specific, since the response to unrelated third party alloantigen-expressing monocytes in unchanged (open bars).

In another set of experiments (Figure 7) it is shown that also a combination of blocking CD40 with the antagonistic anti-CD40 monoclonal antibody 5D12 and blocking of CD80 and CD86 with antagonistic monoclonal antibodies, results in alloantigen-specific T cell unresponsiveness when tested in MLC experiments using PBMCs as detailed above in the materials and methods section. Surprisingly, alloantigen-specific T cell unresponsiveness was also induced when the anti-CD40 monoclonal antibody was combined with the antagonistic anti-CD86 monoclonal antibody without blocking the CD80 costimulatory receptor. In contrast, the combination of the anti-CD40 monoclonal antibody and the anti-CD80 monoclonal antibody, without blocking the CD86 receptor resulted only in T cell hypo-responsiveness to the same level as with the antagonistic anti-CD40 monoclonal antibody alone. This was surprising, since it has extensively been demonstrated that blocking of both CD80 and CD86 always results in more complete inhibition that with either alone. This demonstrates that blocking the CD86-CD28 costimulatory interaction together with blocking the CD40L-CD40 costimulatory interaction with one therapeutic molecule such as described in the above examples has a strong potential for immunotherapy of T cell mediated diseases.

## Claims

1. A soluble molecule capable of binding to the human CD40 antigen by containing at least one antigen-binding site of an antibody to CD40 and capable of binding to the human CD86 antigen by containing at least one antigen-binding site of an antibody to CD86, said antigens being located on the surface of human lymphocytes or antigen-presenting cells .

2. A molecule according to claim 1, which is an antibody containing an antigen-binding site of an antibody to CD40 and an antigen-binding site of an antibody to CD86.

3. A molecule according to claim 1, which is also capable of binding to CD80, containing the antigen-binding site of an antibody which is cross-reactive with CD80 and CD86.

4. A molecule according to claim 1, which is a bispecific diabody capable of binding to human CD40 and to human CD86.

5. A molecule according to claim 1, which is also capable of binding to CD80, containing the antigen-binding site of an antibody to CD80 and the antigen-binding site of an antibody to CD86.

6. A molecule according to claim 1 or 3, which is capable of binding to at least CD86 by means of the extracellular domain of human CTLA-4.

7. A molecule according to any one of claims 1-5, wherein the antibody to CD86 is the antibody Fun-1.

8. A molecule according to any one of claims 1-7, wherein the antibody to CD40 is an antagonistic antibody to CD40.

9. A molecule according to any one of claims 1-7, wherein the antibody to CD40 is a non-stimulatory antagonistic antibody to CD40.

10. A recombinant vector comprising the nucleotide sequences encoding the binding molecule fragments according to any one of claims 1-9, operably linked to regulating sequences capable of expressing the antibody molecule in a host cell.

11. A host cell stably transformed with the vector according to claim 10.

12. A method of producing a recombinant molecule capable of binding to the human CD40 antigen and to at least the human CD86 antigen, comprising culturing the host cell according to claim 11 and isolating the binding molecule from the culture medium.

13. A pharmaceutical composition for induction of T cell tolerance, containing a therapeutically effective amount of the binding molecule according to any one of claims 1-9 and a pharmaceutically acceptable carrier.

14. Use of a binding molecule according to any one of claims 1-9 for preparing a pharmaceutical composition for the treatment of T cell mediated immune responses, for the prevention of allograft transplant or xeno-transplant rejection, for the induction of T cell tolerance, for the induction of allo-transplant or xeno-transplant tolerance, for the prevention or treatment of autoimmune diseases such as rheumatoid arthritis, multiple sclerosis and psoriasis, or for the treatment of T cell mediated immune responses to gene therapy vectors or vehicles or to therapeutic molecules.

## Patentansprüche

1. Ein lösliches Molekül, welches fähig ist, humanes CD40 Antigen zu binden, indem es wenigstens eine Antigenbindungsstelle eines Antikörpers gegen CD40 enthält und welches fähig ist, humanes CD86 Antigen zu binden, indem es wenigstens eine Antigenbindungsstelle eines Antikörpers gegen CD86 enthält, wobei sich die genannten Antigene auf der Oberfläche von humanen Lymphozyten oder Antigenpräsentierenden Zellen befinden.

2. Ein Molekül gemäß Anspruch 1, welches ein Antikörper ist, der eine Antigenbindungsstelle eines Antikörpers gegen CD40 und eine Antigenbindungsstelle eines Antikörpers gegen CD86 enthält.

3. Ein Molekül gemäß Anspruch 1, welches außerdem fähig ist, CD80 zu binden und welches die Antigenbindungsstelle eines Antikörpers enthält, der mit CD80 und CD86 kreuzreagiert.

4. Ein Molekül gemäß Anspruch 1, welches ein bispezifischer Diabody ist, der fähig ist, humanes CD40 und humanes CD86 zu binden.

5. Ein Molekül gemäß Anspruch 1, welches außerdem fähig ist, CD80 zu binden und welches die Antigenbindungsstelle eines Antikörpers gegen CD80 und die Antigenbindungsstelle eines Antikörpers gegen CD86 enthält.

6. Ein Molekül gemäß Anspruch 1 oder 3, welches fähig ist, wenigstens CD86 mittels der extrazellulären Domäne von humanem CTLA-4 zu binden.

7. Ein Molekül gemäß einem der Ansprüche 1-5, worin der Antikörper gegen CD86 der Antikörper Fun-1 ist.

8. Ein Molekül gemäß einem der Ansprüche 1-7, worin der Antikörper gegen CD40 ein antagonistischer Antikörper gegen CD40 ist.

9. Ein Molekül gemäß einem der Ansprüche 1-7, worin der Antikörper gegen CD40 ein nicht-stimulatorischer antagonistischer Antikörper gegen CD40 ist.

10. Ein rekombinanter Vektor, welcher die für die bindenden Molekülfragmente gemäß einem der Ansprüche 1-9 kodierenden Nucleotidsequenzen, operativ verbunden mit Regulationssequenzen, die fähig sind, das Antikörpermolekül in einer Wirtszelle zu exprimieren, umfasst.

11. Eine Wirtszelle, welche stabil mit dem Vektor gemäß Anspruch 10 transformiert ist.

12. Ein Verfahren zur Herstellung eines rekombinanten Moleküls, welches fähig ist, humanes CD40 Antigen und wenigstens humanes CD86 Antigen zu binden und welches die Kultivierung der Wirtszelle gemäß Anspruch 11 und die Isolierung der bindenden Moleküle aus dem Kulturmedium umfasst.

13. Eine pharmazeutische Zusammensetzung für die Induktion von T-Zelltoleranz, welche eine therapeutisch wirksame Menge des bindenden Moleküls gemäß einem der Ansprüche 1 bis 9 und einen pharmazeutisch annehmbaren Träger enthält.

14. Verwendung eines bindenden Moleküls gemäß einem der Ansprüche 1-9 zur Herstellung einer pharmazeutischen Zusammensetzung für die Behandlung von T-Zell vermittelten Immunantworten, für die Prävention der Allotransplantat- oder Xenotransplantat-Abstoßung, für die Induktion von T-Zelltoleranz, für die Induktion von Allotransplantat- oder Xenotransplantat-Toleranz, für die Prävention oder Behandlung von Autoimmunkrankheiten wie z.B. rheumatoide Arthritis, multiple Sklerose und Psoriasis, oder für die Behandlung von T-Zell vermittelten Immunantworten gegenüber Gentherapievektoren oder -vehikeln oder gegenüber therapeutischen Molekülen.

## Revendications

1. Molécule soluble capable de se lier à l'antigène CD40 humain en contenant au moins un site de liaison à un antigène d'un anticorps dirigé contre CD40 et capable de se lier à un antigène CD86 humain en contenant au moins un site de liaison à un antigène d'un anticorps dirigé contre CD86, lesdits antigènes étant situés à la surface de lymphocytes ou de cellules présentatrices d'antigènes humains.

2. Molécule selon la revendication 1, qui est un anticorps contenant un site de liaison à un antigène d'un anticorps dirigé contre CD40 et un site de liaison à un antigène d'un anticorps dirigé contre CD86.

3. Molécule selon la revendication 1, qui est également capable de se lier à CD80, contenant le site de liaison à un antigène d'un anticorps réagissant de façon croisée avec CD80 et CD86.

4. Molécule selon la revendication 1, qui est un dianticorps bispécifique capable de se lier à CD40 humain et à CD86 humain.

5. Molécule selon la revendication 1, qui est également capable de se lier à CD80, contenant le site de liaison à un antigène d'un anticorps dirigé contre CD80 et le site de liaison à un antigène d'un anticorps dirigé contre CD86.

6. Molécule selon la revendication 1 ou 3, qui est capable de se lier au moins à CD86 au moyen du domaine extracellulaire de CTLA-4 humain.

7. Molécule selon l'une des revendications 1 à 5, dans laquelle l'anticorps dirigé contre CD86 est l'anticorps Fun-1.

8. Molécule selon l'une des revendications 1 à 7, dans laquelle l'anticorps dirigé contre CD40 est un anticorps antagoniste dirigé contre CD40.

9. Molécule selon l'une des revendications 1 à 7, dans laquelle l'anticorps dirigé contre CD40 est un anticorps antagoniste non stimulant dirigé contre CD40.

10. Vecteur recombinant comprenant les séquences nucléotidiques codant pour les fragments des molécules de liaison selon l'une des revendications 1 à 9, liées de manière opérationnelle aux séquences régulatrices capables d'exprimer la molécule d'anticorps dans une cellule hôte.

11. Cellule hôte transformée de manière stable par le vecteur selon la revendication 10.

12. Procédé de production d'une molécule recombinante capable de se lier à l'antigène CD40 humain et au moins à l'antigène CD86 humain, comprenant la culture de la cellule hôte selon la revendication 11 et l'isolement de la molécule de liaison à partir du milieu de culture.

13. Composition pharmaceutique pour induire la tolérance des lymphocytes T, contenant une quantité thérapeutiquement active de la molécule de liaison selon l'une des revendications 1 à 9 et un véhicule pharmaceutiquement acceptable.

14. Utilisation d'une molécule de liaison selon l'une des revendications 1 à 9, pour la préparation d'une composition pharmaceutique destinée au traitement des réponses immunitaires conduites par des lymphocytes T, à la prévention du rejet de transplantations d'allogreffes ou de xéno-transplantations, à l'induction de la tolérance des lymphocytes T, à l'induction de la tolérance aux allo-transplantations ou aux xéno-transplantations, à la prévention ou au traitement de maladies auto-immunes, telles que l'arthrite rhumatoïde, la sclérose en plaque et le psoriasis, ou au traitement des réponses immunitaires conduites par des lymphocytes T dirigées contre des vecteurs ou des véhicules de thérapie génique ou contre des molécules thérapeutiques.
